# EUROPEAN PATENT APPLICATION

(11) **EP 2 044 942 A1**
(43) Date of publication of application: **08.04.2009**
(21) Application number: 08020987.7
(22) Date of filing: 20.11.2004
(51) Int. Cl.: A61K 31/565, A61K 31/567, A61K 31/585, A61P 15/00

(54) **Pharmaceutical preparation for continuous hormonal treatment over a period of 21-28 days comprising two ethinylestradio or estradio and drospirenone compositions**

(62) Divisional of application: 04803349.2
(71) Applicant: Bayer Schering Pharma Aktiengesellschaft, 13353 Berlin (DE)
(72) Inventor: Duesterberg, Bernd, 16727 Oberkrämer/OT Bärenklau (DE); Endrikat, Jan, Oakville Ontario L6K 2Y8 (CA); Schuermann, Rolf, 14513 Teltow (DE)

(57) **Abstract**

A pharmaceutical preparation to obtain a continuous hormonal treatment over a desired period of time longer than 21-28 days comprising a first composition containing at least one estrogen and/or at least one progestin in a predetermined amount to be administered in the first 21-28 days and a second composition which contains at least one estrogen and/or at least one progestin in a predetermined amount higher than the amount of the first composition and comprises a mono or multiphase sequence of pharmaceutical dosages.

## Description

### Technical field

The present invention refers to an extended use of a Pharmaceutical preparation comprising estrogens and progestins where a progressive increase of the estrogens and/or progestins dosage after the typical 28 day time period achieves a continuos stimulation of the endometriums. This leads to the desired endometrial stability and to a bleeding free extended regimen.

More particularly the present invention refers to a pharmaceutical preparation to obtain a continuous hormonal treatment over a desired period of time longer than 21-28 days comprising a first composition containing at least one estrogen and/or at least one progestin in a predetermined amount to be administered in the first 21-28 days and a second composition containing at least one estrogen and/or at least one progestin in a predetermined amount higher than the amount of the first composition and which comprises a mono or multiphase sequence of pharmaceutical dosages.

Furthermore the present invention refers to a method of inhibiting ovulation in a mammal, in particular a human, comprising administering to said mammal a first composition containing a certain amount of an estrogen alone or in combination with a progestin for 21-28 days followed by a further administration of a second composition containing an estrogen and a progestin to obtain a continuous hormonal treatment over a period of time longer than 21-28 days. The second composition contains an amount of estrogen and progestin which is higher than the amount of the first composition. The second composition further comprises mono or multiphase sequences of pharmaceutical dosages.

The use of a pharmaceutical preparation for the manufacture of an agent for inhibiting ovulation in a mammal, in particular a human, for inducing long term amenorrhocaand for diminishing symptoms related to hormonal withdrawal is also an aim of the present invention.

The present invention further refers to a pharmaceutical package to be used in a pharmaceutical treatment with an extended regimen.

### Background of the invention

Pharmaceutical contraceptive preparations containing a large number of hormonal components were developed with regard to their suitability in very different administration schemes.

A classification divides the common contraceptive preparations on the market in two general groups. The first one refers to contraceptives containing a constant amount of estrogen and progestin prepared for example in the form of 21 tablets with the combination of active agents and 7 tablets with no active agent. The amount of active agent is the same in each tablet. They are known as monophasic preparation.

Undesired effects related to these kind of pills depend to some extent on the balance between the estrogen and the progestin.

The second group of contraceptives comprises preparations in which the levels of estrogen or progestin vary over the time. According to the number of hormonal levels biphasic or polyphasic preparations are obtained. A typical pattern for this group of contraceptives comprises an estrogen and progestin administration in which a relatively dominant estrogenic formulation is given at the beginning of the treatment cycle with an increasing progestogenic activity towards the end. This administration pattern seems to achieve a better endometrial stability limiting breakthrough bleeding or spotting due to poor epithelialisation of the endometrium.

Contraceptive reliability is mainly provided by the progestin component administered during the treatment. Therefore at least the minimum progestin dosage to effectively inhibit ovulation should be provided with a daily dosage. On the other hand the estrogen increases the ovulation inhibitory effects of progestin and ensures cycle stability.

Typical regimen for hormonal contraceptive treatments resemble the natural course of the cycle with an administration-free interval of about 7 days whereby withdrawal bleeding simulates the natural menses. Thus, as mentioned above, 21 day intervals of hormone administration alternate with 7 days during which no hormones are administered.

Since the psychological and physical ability in women during the premenstrual phase is subject to limitations, in several circumstances a delay of the menses is our days often required. Typical cases are for example sport competitions, particular medical exams or certain travel situations.

A delay of menstruation in women treated with an hormonal preparation like those previously mentioned is easily affordable as reported in the literature ("Kontrazeption mit Hormonen: ein Leitfaden für die Praxis", Hans-Dieter Taubert and Herbert Kuhl, 2nd edition 1995, Georg Thieme Verlag , pp. 199-201).

By monophasic preparation for example it is possible to achieve said delay simply starting on the 22^{nd} day of treatment a new kit of contraception pills and maintain the treatment for the desired period of time up to 2-3 days before the desired withdrawal bleeding. Only the dose of the last phase should he applied for a delay of the menstruation when polyphasic hormonal preparations arc used.

In general with the methods previously mentioned a withdrawal bleeding delay of at least 7 days can be easily obtained.

The hormonal treatment can be directed not only to prevent pregnancy but also to avoid all derived symptoms related to hormonal withdrawal like for example premenstrual symptoms, dysmenorrhea, endometriosis, menstrual migraine and acne.

In these cases a longer inhibition of the ovulation obtained through the hormonal contraception would allow to overcome problems related to hormonal changes revealing to be well accepted and particularly effective over a three month period (84 days).

Such a treatment longer then 21-28 is also known as extended use regimen.

WO03/049744 describes female oral contraceptives that prevent pregnancy and treat premenstrual syndrome (PMS) including premenstrual dysphoric disorder (PMDD). Said document further describes a method of preventing pregnancy and treating PMS including PMDD, by avoiding complete withdrawal of estrogen at the end of the treatment period, or between treatment periods, by administering oral contraceptives in an extended regimen without a break. According to the above document premenstrual symptoms are rare when menstruation is infrequent, for this reason users of oral contraceptives would have lower rates of premenstrual symptoms, when exposed to peaks and troughs of endogenous progesterone with a protective effect against PMDD.

WO03/049744 further describes a method of preventing pregnancy, which involves administering one or two combination regimens of oral contraceptive. The hormonal treatment is applied for 110 consecutive days.

The main drawbacks of the method reported in WO03/049744 concern the variation of the hormonal levels during the extended treatment. Increasing and decreasing of estrogen and progestin dosages determine instability of the endometrium with an increasing of side effects like breakthrough bleeding.

An altered standard 21-day/7-day oral contraceptive regimen with the use of a monophasic pill with 30 to 35 µg of ethinylestradiol and different type of progestins to delay menses and reduce hormone withdrawal symptoms is also known in the art (PJ Sulak et al., Am J Obstet Grynecol, 2002; 186: 1142-1149). Also in this case one of the main side effects relates to a high incidence of breakthrough bleeding, which is a common cause for discontinuation of the regimen.

WO99/09993 describes an oral contraception regimen which comprises sequentially administering two or more progestational agents exhibiting different effects on the human endometrium in combination with an estrogen. Particularly an extended use of an oral contraception regimen is reported which comprises the sequential administration of two or more progestational agents in combination with an estrogen. Once again the extended regimen is based on the modulation of the progestational agent with a waving pattern while the estrogen dose remains constant.

Extended use regimens would be clearly desirable both to inhibit ovulation and to offer freedom from menstrual flow and premenstrual symptoms for extended periods of time. Such extended use regimens would also have a favourable therapeutic use in premenstrualtype symptoms, dysmenorrhea, menstrual migraine. The continuos use of an oral contraceptive can also have a favourable effect on acne since acne typically reoccurs during the pill free period. Such a method particularly for the intended long bleeding free period should not present the incidence of bleeding abnormalities such as spotting or breakthrough bleeding while exhibiting a favourable effect on endomentrial morphology.

### Description of the invention

The present invention aims to set aside the drawbacks related to the known art and particularly aims to provide a pharmaceutical preparation to obtain a continuous hormonal treatment over a desired period of time longer than 21-28 days comprising a first composition containing at least one estrogen and/or at least one progestin in a predetermined amount to be administered in the first 21-28 days and a second composition comprising at least one estrogen and/or at least one progestin in a predetermined amount higher than the amount of the first composition and also comprising a mono or multiphase sequence of pharmaceutical dosages.

The use of a pharmaceutical preparation for the manufacture of an agent for inhibiting ovulation in a mammal, in particular a human and for diminishing symptoms related to hormonal withdrawal is also a goal of the present invention.

A further aim of the present invention is to provide a method of inhibiting ovulation in a mammal, in particular a human, comprising administering to said mammal a first composition containing at least one estrogen and/or at least one progestin in a predetermined amount for 21-28 days comprising a further administration of a second composition containing at least one estrogen and at least one progestin in a predetermined amount performed to obtain a continuous hormonal treatment over a desired period of time longer than 21-28 days. The second composition contains at least one estrogen and/or at least one progestin in a predetermined amount which is higher than the amount of the first composition and comprises a mono or multiphase sequence of pharmaceutical dosages.

Said method can be further advantageously used in hormonal withdraw related syndrome for diminishing premenstrual symptoms, dysmenorrhea, endometriosis, menstrual migraine. The continuos use of an oral contraceptive according to the above method have also a favourable effect on acne since acne typically reoccurs during the pill free period.

The present invention further refers to pharmaceutical package for an extended regimen treatment longer than 21-28 days comprising a first composition containing at least one estrogen and/or at least one progestin in a predetermined amount to be administered in the first 21-28 days and a second composition containing at least one estrogen and/or at least one progestin in a predetermined amount higher than the amount of the first composition to be administered in the following of the treatment and comprising a mono or multiphase sequence of pharmaceutical dosages.

The concept of a prolonged hormonal treatment to inhibit ovulation, for example over a three month time period and with a constant stepwise increase of the hormonal dosage originated from the observation that during pregnancy bleeding abnormalities are seldom observed. Furthermore, during gestation the hormonal levels of estrogen and progestin gradually increase with the time.

According to the present invention, a progressive increase of the estrogen and/or progestin dosage allows a continuos and stepwise increasing stimulation of the endometrium, which leads to the desired stability of the endometrium and bleeding free extended regimen.

The pharmaceutical preparation to obtain a continuous hormonal treatment over a desired period of time longer than 21-28 days comprises, according to the present invention, a first composition containing at least one estrogen and/or at least one progestin in a predetermined amount to be administered in the first 21-28 days. To said first composition follows a second composition comprising at least one estrogen and/or at least one progestin in a predetermined amount higher than the amount of the first composition and also comprising a mono or multiphase sequence of pharmaceutical dosages.

More in detail, in the second composition of the pharmaceutical preparation only the amount of estrogen or only the amount of progestin or both the amount of estrogen and progestin are higher than the respective amount comprised in the first composition.

The estrogen used in the first and/or in the second composition can be at least one synthetic estrogen, at least one biogenic estrogen or mixtures thereof.

According to the invention said synthetic estrogen can be selected from the group consisting of: ethinylestradiol, mestranol, quinestranol, precursors capable of liberating such an estrogen when used in the present pharmaceutical preparation and mixtures thereof Most preferably the synthetic estrogen is ethinylestradiol or a precursor capable of liberating ethinylestradiol. The daily hormone units for use during the whole extended treatment preferably contain the synthetic estrogen in an amount equivalent to 0.005-0.050 mg of ethinylestradiol, most preferably in an amount equivalent to 0.005-0.030 mg of ethinylestradiol.

The biogenic estrogen is preferably selected from the group consisting of: estradiol, estrone, estran, estriol, estetrol, conjugated equine estrogens, precursors capable of liberating such an estrogen when used in the present pharmaceutical preparation and mixtures thereof. Most preferably the biogenic estrogen is estradiol or a precursor capable of liberating estradiol, the term estradiol encompassing 17beta-estradiol. Most preferably the biogenic estrogen is estradiol or a precursor thereof.

The daily oral hormone units for use during the whole extended treatment preferably contain the biogenic estrogen in an amount equivalent to 0.1-5 mg of estradiol, most preferably in an amount equivalent to 0.1-3.0 mg of estradiol.

The progestin contained in the pharmaceutical preparation according to the invention is preferably selected from the group consisting of levonorgestrel, norgestimate, norethisterone, dydrogestcronc, drospirenonc, 3-beta-hydroxydesogestrel, 3-keto desogestrel (=etonogestrel), 17-deacctyl norgestimate, 19-norprogesterone, acetoxypregnenolone, allylestrenol, anagestone, chlormadinone acetate, cyproterone acetate, demegestone, desogestrel, dienogest, dihydrogesterone, dimethisterone, ethisterone, ethynodiol diacetate, flurogestone acetate, gastrinon, gestodene, gestrinone, hydroxymethylprogesterone, hydroxyprogesterone, lynestrenol (=lynoestrenol), medrogestone, medroxyprogesterone acetate, megestrol, melengestrol, nomegestrol, norethindrone (norethisterone), norethynodrel, norgestrel (includes d-norgestrel and dl norgestrel), norgestrionone, normethisterone, progesterone, quingestanol, (17alpha)-17-hydroxy-11-methylene-19-norpregna-4,15-diene-20-yn-3-one, tibolone, algestone acetophenide, nestorone, promegestone, 17-hydroxyprogesterone esters, 19-nor-17hydroxyprogesterone, 17alpba-ethinyl-testosterone, 17alpha-ethinyl-19-nor-testosterone, d-17beta-aeetoxy-13beta-ethyl-17alpha-ethinyl-gon-4-en-3-one oxime, hydroxytriendione ((21S)-21-hydroxy-21-methyl-14,17ethano-19-nor-pregna4,9,15-triene-3,20-dione), 5-{2-hydroxy-3-[1-(2-fluoro-5-trifluromethylphenyl)-cyclopropyl]-2-trifluoromethyl-propionylamino}-phthalide and precursors of these compounds.

Specific examples of progestin precursor which may be employed in accordance with the present invention comprise: anagestone acetate, gestodene acetate, hydroxymethylprogesterone acetate, hydroxyprogesterone acetate, hydroxyprogesterone hexanoate, hydroxyprogesterone caproate, hydroxyprogesterone enanthate, megestrol acetate, melengestrol acetate, nomegestrol acetate, norethindrone acetate, norethisterone acetate, norethisterone enanthate, quingestanol acetate, (17alpha)-17-hydroxy- 11-methytene-19-norpregna-4,15-diene₋20-yn-3-one, tibolone, algestone acetophenide, nestorone, promegestone, 17-hydroxyprogesterone esters, 19-nor-17hydroxyprogesterone esters, 17alpha-ethinyl-testosterone.

Preferably the progestin is selected from the group consisting of levonorgestrel, dienogest, gestodene, drospirenone, and precursors thereof. The daily hormone units for use during the whole extended treatment preferably contain the at least one progestin in an amount of 0.03-0.25 mg of levonorgestrel and/or 0.5-5 mg of dienogest and/or 0.03-0.15 mg of gestodene, and/or 0.5-5 mg of drospirenone or equivalent dosages of other progestins.

Best results arc obtained with the pharmaceutical preparation according to the invention when the estrogen is ethinylestradiol and/or estradiol or a precursor thereof and the progestin is selected from the group consisting of levonorgestrel, dienogest, gestodene, drospirenone, and their precursors.

Thus in a preferred embodiment the pharmaceutical preparation to obtain a continuous hormonal treatment over a desired period of time longer than 21-28 days comprises, according to the present invention, a first composition containing ethinylestradiol and/or estradiol or precursors thereof in a therapeutically effective amount to inhibit ovulation and/or a progestin preferably selected from the group consisting of levonorgestrel, dienogest, gestodene, drospirenone, and their precursors in a predetermined therapeutically effective amount to be administered in the first 21-28 days. To said first composition follows a second composition comprising at least one estrogen and/or at least one progestin both selected from the group estrogen and progestin previously mentioned in a predetermined therapeutically effective amount higher than the amount of the first composition.

According to a particularly favourable embodiment of the present invention the second composition comprises a mono or multiphase sequence of pharmaceutical dosages so that the estrogen and progestin hormonal concentration can be gradually increased over the time.

The hormonal dosages of estrogen and progestin previously reported refer particularly to oral formulations. The formulation of an estrogen and progestin for the preparation of a combined preparation according to the invention is effected in a manner completely analogous to that already known for conventional oral contraceptives having a 21-day administration period of active ingredients, such as, for example, Femovan^{®}, Meliane^{®} and Mirelle^{®} (ethinylestradiol/gestodene), or Miranoya^{®} and Microgynon^{®} (ethmylestradiol/levonorgestrel), or Yasmin^{®} and Yaz^{®} (ethinylestradiol/drospirenonc) or Valette^{®} (ethinylestradiol/dienogest).

The formulation of the exclusively progestin-containing unit doses can also be carried out in a completely analogous manner to that known for progestin-containing agents designed for oral administration that are already available, for example Microlut^{®} with 0,03 mg LN.

The unit doses of the first composition of the pharmaceutical preparation according to the invention may also be in the form of a plaster (transdermal application), an implant, a vaginal ring or another depot formulation and thus administered continuously.

The hormone units according to the invention may be also administered parenterally for example in an daily amount of 0.01-0.10 mg estradiol or equivalents of other biogenic estrogens, sublingually, transdermally, intravaginally, intranasally or buccally. The daily hormonal units can suitably be administered orally, transdermally or intravaginally.

For such administration which avoids a first pass effect lower hormone units are administered, for example lower dosages of biogenic estrogens equivalent to 0.005-0.030 mg of ethinylestradiol can be used.

A particularly favourable form of embodiment of this invention refers to a preparation for transdermal administration for example in the form of a plaster comprising said first or second composition suitable formulated therein, for example dissolved in a convenient percentage in a non-flowable, physiologically acceptable gel that is microdispersed in a cross-linked silicone elastomer.

In order to determine equivalently effective amounts of ethinyloestradiol and estradiol on the one hand and of different progestins, such as levonorgestrel, dienogest, gestodene and drospirenone, on the other hand, reference is made to the data given in EP-A-0 253 607. Further details for determining dosage equivalents of different progestogenic active ingredients may be found, for example, in "Probleme der Dosistindung: Sexualhormone" (Problems of dosage determination: sexual hormones); F. Neumann et al. in "Arzncimittelforschung" (Drug Research) 27, 2a, 296-318 (1977) and in "Aktuelle Entwicklungen in der Hormonalen Kontrazeption" (Current developments in hormonal contraception), H. Kuhl in "Gynakologe" 25: 231-240 (1992).

According to the present invention the pharmaceutical preparation can comprise pharmaceutical dosages for the administration over a total time of 56, 84, 112 or 140, 168 days. The preferred administration regimen cover a total time of 84 and 112 days.

Between the administration of two sequential pharmaceutical preparations, a free hormonal administration period of 1-7 days can be provided. Said period can be accordingly to a particular form of embodiment of the invention of 4-7 days.

The present invention further relates to a method of inhibiting ovulation in a mammal, in particular a human, comprising administering to said mammal said first composition containing at least one estrogen and/or at least one progestin in a predetermined amount for 21-28 days and a further administration of said second composition containing at least one estrogen and at least one progestin in a predetermined amount performed to obtain a continuous hormonal treatment over a desired period of time longer than 21-28 days.

Said second composition as previously described contains at least one estrogen and/or at least one progestin in a predetermined amount which is higher than the amount of the first composition and comprises a mono or multiphase sequence of pharmaceutical dosages.

The method according to this invention can be further advantageously used for diminishing symptoms related to hormonal withdrawal such as premenstrual symptoms, dysmenorrhes, endometriosis and menstrual migraine. The continuos use of an oral contraceptive can also have a favourable effect on acne since acne typically reoccurs during the pill free period.

The method according to the present invention with a stepwise increase of the hormonal intake exhibits an optimum combination of contraceptive reliability, cycle control and minimum side-effects along the whole extended regimen.

According to a particular form of embodiment of the method according to this invention the user of this particular extended regimen starts the intake of the lowest dose on the first day of menstruation. Said lowest dose corresponds to the said first composition. The individual takes this first dose uninterruptedly until experiencing early signs of spotting (e.g., brown vaginal discharge) normally for at least 28 days. On this day of first signs of spotting the user switches to intake of the next higher dosage form corresponding to said second composition or according to a particular embodiment of this invention to one of the multiphase sequences of said second composition.

The user follows this stepwise increasing intake schedule until spotting during intake of the highest dosed tablets occurs. At this point the intake period comes to an end and a 7-day free interval will allow withdrawal bleeding. After this week the treatment period would start again.

A pharmaceutical package for an extended regimen treatment longer than 21-28 days is also part of the present invention and comprises:
- a first composition containing at least one estrogen and/or at least one progestin in a predetermined amount to be administered in the first 21-28 days
- a second composition containing at least one estrogen and/or at least one progestin in a predetermined amount higher than the amount of the first composition to be administered in the following of the treatment and comprising a mono or multiphase sequence of pharmaceutical dosages.

A particularly favourable form of embodiment of the invention comprises as a form of packing for the pharmaceutical preparation a blister; other forms of packing known for that purpose are, however, also possible.

### Description of some form of embodiment

Other features and advantages of the invention will become apparent from the following description of some favourable form of embodiment, provided purely as a non-restricting examples:

### Example 1

The following clinical study is performed to examine the efficacy of the suggested step-up dose regimens for the extended use of hormonal contraception. The study is designed to test the hypothesis that a phasic regimen with increasing dosages, for example of estrogens and progestins, results in bleeding free intervals of 84 days while maintaining the contraceptive protection. In the present study two different phasic regimens with a conventional 21 day regimen based on the number of bleeding days during an observation period of 84 days are considered.

The number and type of adverse events arc compared and the number of pregnancies are registered as well.

A three arms, multicenter, randomised, open study is designed with a study population with young fertile women of 18-35 having regular menstrual cycles. Exclusion criteria comprised contraindications for hormonal contraceptives.
Test treatments (EE= ethinylestradiol, DRSP= drospirenone)

| | |
|---|---|
| Preparation A | 0.015mg EE plus 2.0mg DRSP for 28 days |
| | 0.020mg EE plus 2.5mg DRSP for 28 days |
| | 0.030mg EE plus 3.0mg DRSP for 28 days |
| | |
| Preparation B | 0.020mg EE plus 2.0mg DRSP for 28 days |
| | 0.020mg EE plus 2.5mg DRSP for 28 days |
| | 0.020mg EE plus 3.0mg DRSP for 28 days |
| | |
| Preparation C (reference) | 0.020mg EE plus 3.0mg DRSP for 21 days plus 7 days placebo |
| | 0.020mg EE plus 3.0mg DRSP for 21 days plus 7 days placebo |
| | 0.020mg EE plus 3.0mg DRSP for 21 days plus 7 days placebo |

The primary efficacy variable is the number of bleeding days (slight and heavy bleeding) during the whole observation period of 84 days.
The secondary efficacy variable is the number of adverse events and pregnancies.
The number of subjects is determined biometrically with roughly about 200 subjects per test group.

### Example 2

An other clinical study is performed according to the protocol of example 1 but with the following test treatment (EE= ethinylestradiol, GSD= gestodene):

| | |
|---|---|
| Preparation A | 0.015mg EE plus 0.060mg GSD for 28 days |
| | 0.020mg EE plus 0.075mg GSD for 14 days |
| | 0.030mg EE plus 0.075mg GSD for 14 days |
| | 0.030mg EE plus 0.100mg GSD for 14 days |
| | |
| Preparation B | 0.030mg EE plus 0.060mg GSD for 28 days |
| | 0.030mg EE plus 0.075mg GSD for 28 days |
| | 0.030mg EE plus 0.100mg GSD for 28 days |
| | |
| Preparation C (ref.) | 0.015mg EE plus 0.060mg GSD for 24 days plus 4 days placebo |
| | 0.015mg EE plus 0.060mg GSD for 24 days plus 4 days placebo |
| | 0.015mg EE plus 0.060mg GSD for 24 days plus 4 days placebo |

### Example 3

An other clinical study is performed according to the protocol of example 1 but with the following test treatment (EE= ethinylestradiol, LNG= levonorgestrel):

| | |
|---|---|
| Preparation A | 0.020mg EE plus 0.080mg LNG for 28 days |
| | 0.030mg EE plus 0.100mg LNG for 28 days |
| | 0.030mg EE plus 0.125mg LNG for 28 days |
| | 0.030mg EE plus 0.150mg LNG for 28 days |
| | |
| Preparation B | 0.030mg EE plus 0.080mg LNG for 28 days |
| | 0.030mg EE plus 0.100mg LNG for 28 days |
| | 0.030mg EE plus 0.125mg LNG for 28 days |
| | 0.030mg EE plus 0.150mg LNG for 28 days |
| | |
| Preparation C (ref.) | 0.020mg EE plus 0.100mg LNG for 21 days plus 7 days placebo |
| | 0.020mg EE plus 0.100mg LNG for 21 days plus 7 days placebo |
| | 0.020mg EE plus 0.100mg LNG for 21 days plus 7 days placebo |
| | 0.020mg EE plus 0.100mg LNG for 21 days plus 7 days placebo |

### Example 4

A further clinical study is performed according to the protocol of example 1 but with the following test treatment (EE= ethinylestradiol, DNG= dienogest):

| | |
|---|---|
| Preparation A | 0.010mg EE plus 1.50mg DNG for 28 days |
| | 0.015mg EE plus 2.00mg DNG for 28 days |
| | 0.020mg EE plus 2.50mg DNG for 28 days |
| | 0.030mg EE plus 2.50mg DNG for 28 days |
| | |
| Preparation B | 0.010mg EE plus 2.00mg DNG for 28 days |
| | 0.015mg EE plus 2.00mg DNG for 28 days |
| | 0.020mg EE plus 2.00mg DNG for 28 days |
| | 0.030mg EE plus 2.00mg DNG for 28 days |
| | |
| Preparation C (rcf.) | 0.030mg EE plus 2.00mg DNG for 21 days plus 7 days placebo |
| | 0.030mg EE plus 2.00mg DNG for 21 days plus 7 days placebo |
| | 0.030mg EE plus 2.00mg DNG for 21 days plus 7 days placebo |
| | 0.030mg EE plus 2.00mg DNG for 21 days plus 7 days placebo |

The results deriving from the previous example are compared to those of the reference preparations with the following remarks:
- Lower rate of intermenstrual bleeding, shorter period of bleeding and lower bleeding intensity;
- Lower rate of hormone withdrawal related symptoms;
- Improved women wellbeing.

The invention is described above with reference to preferred forms of embodiment. It is nevertheless clear that the invention is susceptible to numerous variations within the framework of technical equivalents.

### Specific embodiments of the invention:

1. A pharmaceutical preparation to obtain a continuous hormonal treatment over a desired period of time longer than 21-28 days comprising a first composition containing at least one estrogen and/or at least one progestin in a predetermined amount to be administered in the first 21-28 days and a second composition whcih contains at least one estrogen and/or at least one progestin in a predetermined amount higher than the amount of the first composition and comprises a mono or multiphase sequence of pharmaceutical dosages.
2. A pharmaceutical preparation according to embodiment 1 comprising pharmaceutical dosages for the administration over a total time of 56, 84, 112, 140, or 168 days.
3. A pharmaceutical preparation according to embodiments 1 and 2 in which the second composition comprises a one phase sequence of pharmaceutical dosages.
4. A pharmaceutical preparation according to embodiments 1 and 2 in which the second composition comprises a two phase sequence of pharmaceutical dosages.
5. A pharmaceutical preparation according to embodiment 3 in which the pharmaceutical dosage of at least one estrogen of the second composition is higher than the amount contained in the first composition.
6. A pharmaceutical preparation according to embodiment 3 in which the pharmaceutical dosage of at least one progestin of the second composition is higher than the amount contained in the first composition.
7. A pharmaceutical preparation according to embodiment 4 in which the pharmaceutical dosage of at least one estrogen and/or at least one progestin of the second composition are higher than the amount contained in the first composition.
8. A pharmaceutical preparation according to anyone of the previous embodiments 1 to 7 characterised in that the at least one estrogen is selected from the group consisting of following synthetic estrogens: ethinylestradiol, mestranol, guinestranol, or a precursors capable of liberating such an synthetic estrogen and/or from the group of the following biogenic estrogens: estradiol, estrone, cstran, estriol, estetrol, conjugated equine estrogens, precursors capable of liberating such a biogenic estrogen.
9. A pharmaceutical preparation according to embodiment 8 characterised in that the at least one estrogen is ethinylestradiol and/or estradiol.
10. A pharmaceutical preparation according to anyone of the previous embodiments to 9 in which the daily hormone units of estrogen preferably contain ethinylestradiol in an amount of 0.005-50 mg, most preferably in an amount of 0.005-0.030 mg and/or the estradiol in an amount of to 0.1-5.0 mg.
11. A pharmaceutical preparation according to anyone of the previous embodiments 1 to 10 in which the at least one progestin is selected from the group consisting of levonorgestrel, norgestimate, norethisterone, dydrogesterone, drospirenone, 3-beta-hydroxydesogestrel, 3-keto desogestrel (=etonogestrel), 17-deacetyl norgestimate, 19-norprogesterone, acetoxypregnenolone, allylestrenol, anagcstone, chlormadinone acetate, cyproterone acetate, demcgestone, desogestrel, dienogest, dihydrogesterone, dimethisterone, ethisterone, ethynodiol diacetate, flurogestone acetate, gastrinon, gestodene, gestrinone, hydroxymethylprogesterone, hydroxyprogesterone, lynestrenol (-lynoestrenol), medrogestone, medroxyprogesterone acetate, megestrol, melengestrol, nomegestrol, norethindrone (=norethisterone), norethynodrel, norgestrel (includes d-norgestrel and dl norgestrel), norgestrienone, normethisterone, progesterone, quingestanol, (17alpha)-17-hydroxy-11-methylene-19-norpregna-4,15-diene-20-yn-3-one, tibolone, algestone acetophenide, nestorone, promegestone, 17-hydroxyprogesterone esters, 19-nor-17hydroxyprogesterone, 17alpha-ethinyl-testosterone, 17alpha-ethinyl-19-nor-testosterone, d-17beta-acetoxy-13beta-ethyl-17alpha-ethinyl-gon-4-en-3-one oxime, hydroxytriendione ((21S)-21-hydroxy-21-methyl-14,17ethano-19-nor-pregna-4,9,15-triene-3,20-dione), 5-{2-hydroxy-3-[1-(2-fluoro-5-trifluromethylphenyl)-cyclopropyl]-2-trifluoromethyl-propionylamino}-phthalide and precursors thereof.
12. A pharmaceutical preparation according to anyone of the previous embodiments I to 11 in which the at least one progestin is preferably selected from the group consisting of levonorgestrel, dienogest, gestodene, drospirenone, and precursors thereof.
13. A pharmaceutical preparation according to anyone of the previous embodiments 1 to 12 in which the daily hormone units of at least a progestin for use during the whole extended treatment preferably contain the progestin in an amount of 0.03-0.25 mg of levonorgestrel and/or 0.5-5 mg of dienogest and/or 0.03-0.15 mg of gestodene, and/or 0.5-5 mg of drospirenone or equivalent dosages of other progestins.
14. A pharmaceutical preparation according to anyone of the previous embodiment I to 13 in which the hormone units are administered orally, parenterally, sublingually, transdermally, intravaginally, intranasally or buccally.
15. A pharmaceutical preparation according to anyone of the previous embodiments 1 to 14 in which the hormone units are for oral administration.
16. A pharmaceutical preparation according to anyone of the previous embodiments 1 to 15 in which the hormone units are daily units.
17. Use of a pharmaceutical preparation according to embodiments 1 to 16 for the manufacture of an agent for inhibiting ovulation in a mammal, in particular a human.
18. Use of a pharmaceutical preparation according to embodiment 17 in which the administration of said preparation extends over a time of 56, 84, 112, 140 or 168 days.
19. Use of a pharmaceutical preparation according to embodiments 17 to 18 for the manufacture of an agent for diminishing symptoms related to hormonal withdrawal such as premenstrual symptoms, dysmenorrhea, endometriosis, menstrual migraine.
20. Use of a pharmaceutical preparation according to embodiments 17 to 18 for the manufacture of an agent for diminishing symptoms related to acne
21. A pharmaceutical package for an extended regimen treatment longer than 21-28 days comprising:
   - a first composition containing at least one estrogen and/or at least one progestin in a predetermined amount to be administered in the first 21-28 days
   - a second composition containing at least one estrogen and/or at least one progestin in a predetermined amount higher than the amount of the first composition to be administered in the following of the treatment and comprising a mono or multiphase sequence of pharmaceutical dosages.
22. Pharmaceutical package according to embodiment 21 in which said first composition and said second composition correspond to the first and the second composition as defined in the Pharmaceutical preparation according to claim 1 to 16.
23. Pharmaceutical package according to embodiments 21 and 22 in which the first and/or the second composition are administered in daily doses.
24. Pharmaceutical package according to anyone of the embodiments 21 to 23 in which the first and/or the second composition are arranged for separate sequential administration like for example in separate blisters.
25. Pharmaceutical package according to anyone of the embodiments 21 to 24 in which the administration of the first and second composition extends over a time of 56, 84, 112, 140 or 168 days.

## Claims

1. A pharmaceutical preparation to obtain a continuous hormonal treatment over a desired period of time longer than 21-28 days with a constant stepwise increase of the hormonal intake comprising a first composition containing ethinylestradiol or estradiol, and drospirenone in a predetermined amount to be administered in the first 21-28 days and a second composition **characterised in that** it contains ethinylestradiol or estradiol, and drospirenone in a predetermined amount higher than the amount of the first composition and comprises a mono or multiphase sequence of pharmaceutical dosages.

2. A pharmaceutical preparation according to claim 1 **characterised in that** it comprises pharmaceutical dosages for the administration over a total time of 56, 84, 112, 140, or 168 days.

3. A pharmaceutical preparation according to claim 1 and 2 **characterised in that** the second composition comprises a one phase sequence of pharmaceutical dosages.

4. A pharmaceutical preparation according to claim 1 and 2 **characterised in that** the second composition comprises a two phase sequence of pharmaceutical dosages.

5. A pharmaceutical preparation according to claim 3 **characterised in that** the pharmaceutical dosage of ethinylestradiol or estradiol of the second composition is higher than the amount contained in the first composition.

6. A pharmaceutical preparation according to claim 3 **characterised in that** the pharmaceutical dosage of progestin of the second composition is higher than the amount contained in the first composition.

7. A pharmaceutical preparation according to claim 4 **characterised in that** the pharmaceutical dosage of ethinylestradiol or estradiol, and drospirenone of the second composition arc higher than the amount contained in the first composition

8. A pharmaceutical preparation according to anyone of the previous claims **characterised in that** the daily hormone units preferably contain ethinylestradiol or estradiol in an amount of 0.005-50 mg, most preferably in an amount of 0.005-0.030 mg.

9. A pharmaceutical preparation according to anyone of the previous claims **characterised in that** the daily hormone units of at least a progestin for use during the whole extended treatment preferably contain 0.5-5 mg of drospirenone or equivalent dosages of other progestins.

10. A pharmaceutical preparation according to anyone of the previous claims **characterised in that** the hormone units are administered orally, parenterally, sublingually, transdermally, intravaginally, intranasally or buccally.

11. A pharmaceutical preparation according to anyone of the previous claims **characterised in that** the hormone units are for oral administration.

12. A pharmaceutical preparation according to anyone of the previous claims **characterised in that** the hormone units are daily units.

13. Use of a pharmaceutical preparation according to claims 1 to 12 for the manufacture of an agent for inhibiting ovulation in a mammal, in particular a human.

14. Use of a pharmaceutical preparation according to claim 13 **characterised in that** the administration of said preparation extends over a time of 56, 84, 112, 140 or 168 days.

15. A pharmaceutical package for an extended regimen treatment longer than 21-28 days with a constant stepwise increase of the hormonal intake comprising:
- a first composition containing ethinylestradiol or estradiol, and drospirenone in a predetermined amount to be administered in the first 21-28 days
- a second composition containing ethinylestradiol or estradiol, and drospirenone in a predetermined amount higher than the amount of the first composition to be administered in the following of the treatment and comprising a mono or multiphase sequence of pharmaceutical dosages.
